# EUROPEAN PATENT APPLICATION

(11) **EP 3 875 044 A1**
(43) Date of publication of application: **08.09.2021**
(21) Application number: 19878412.6
(22) Date of filing: 01.11.2019
(51) Int. Cl.: A61B 17/11

(54) **PUSH ROD SELF-ADAPTIVE STRUCTURE**

(30) Priority: 02.11.2018 CN 201811302979
(71) Applicant: Ezisurg (Suzhou) Medical Co., Ltd., Suzhou, Jiangsu 215163 (CN)
(72) Inventor: YANG, Jun, Shanghai 201318 (CN); TANG, Chuangang, Shanghai 201318 (CN); LIAO, Menghui, Shanghai 201318 (CN); BAO, Minghui, Shanghai 201318 (CN); NIE, Honglin, Shanghai 201318 (CN)
(74) Representative: Mansfield, Peter Turquand
(86) International application number: PCT/CN2019/114935
(87) International publication number: WO 2020/088628

(57) **Abstract**

The present invention discloses an adaptive drive beam structure, including a plurality of drive beams (1, 2, 3, 4, 5), a central shaft joint (7) and a drive beam joint (6), characterized in that the plurality of drive beams (1, 2, 3, 4, 5) are mounted in the central shaft joint (7) through the drive beam joint (6), and when a staggered front and back occurs among the plurality of drive beams (1, 2, 3, 4, 5), the drive beam joint (6) is capable of compensating the mismatch of the plurality of drive beams through rotation movement, such that the plurality of drive beams (1, 2, 3, 4, 5) are simultaneously in contact with the drive beam joint (6) all the time. The adaptive drive beam structure is applied to rotating type device products, thereby improving the stress of flexible drive beam in a device, ensuring that the plurality of drive beams are stressed uniformly in the movement process, reducing the deformation of a single piece drive beam and ensuring the movement smoothness of the drive beam structure in operation.

## Description

### TECHNICAL FIELD

The present application relates to a self-adaptive structure for a rotating device. The rotating device is an articulating endoscopic stapler. The present application particularly relates to an adaptive drive beam structure in an articulating endoscopic stapler.

### BACKGROUND

The stapler is the main medical device used in medicine for replacing the traditional hand suture, and has the advantages of convenience in use, rapid suturing, tight suturing, moderate tightness and few side effects of surgical complications. The angle of the head end of the articulating endoscopic stapler is rotatable, so the articulating endoscopic stapler can be flexibly applied to many stapling regions, for example, it may be applied to opening or excision, transection and anastomosis of lung, stomach and intestine tissues under the endoscope.

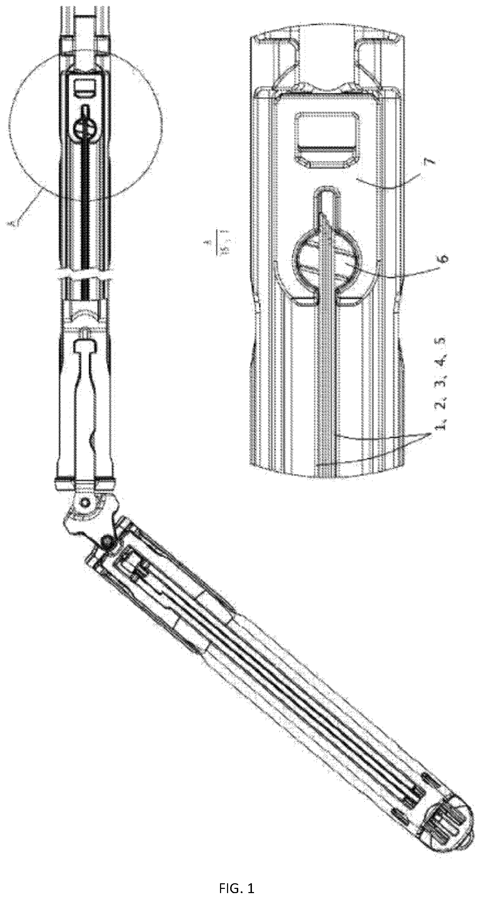

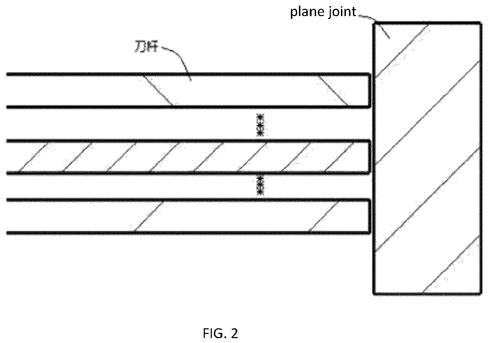

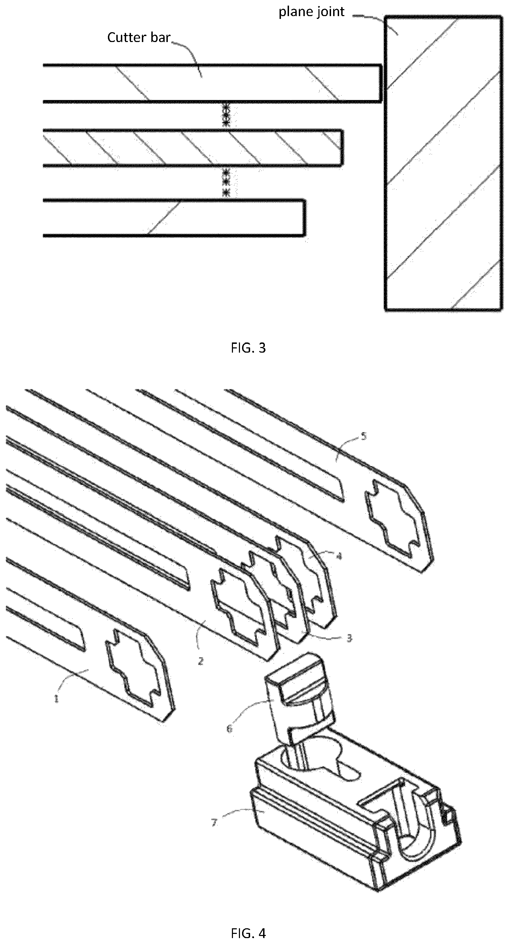

In the rotating type endoscopic stapler, to meet the turning function requirement, the flexible drive beam is an essential part. At present, the joint method of the drive beam in the rotating type endoscopic stapler in the industry is basically the connection of a plurality of drive beam and a plane joint, as shown in FIG. 1. In the rotating type stapler, when the stapler jaw is in a linear state, a plurality of drive beams are simultaneously in contact with the plane joint and simultaneously bear the pushing force from the plane joint, as shown in FIG. 2; however, when the stapler jaw rotates, as shown in FIG. 1, the drive beams will bend and deform, and at this time, due to the difference of inner and outer diameters caused by bending, a staggered front and back will occur among the drive beams, and only one of the plurality of drive beams is in contact with the plane, as shown in FIG. 3, only one drive beam bears the pushing force from the plane joint, such that the drive beam bears larger deformation, the drive beam does not move smoothly in the front and back movement process, and the challenge to the drive beam is higher.

### SUMMARY

The technical problem to be solved by the present application is to overcome the defects in the prior art, by providing a push self-adaptive structure for a rotating type device and with a unique structural design. According to the present application, the stress of the flexible drive beam in the rotating type device can be improved, uniform stress of the plurality of drive beams in the movement process can be ensured, the deformation of a single piece drive beam is reduced, and the movement smoothness of the drive beam structure in operation is ensured.

The present application provides an adaptive drive beam structure, including a plurality of drive beams, a central shaft joint and a drive beam joint, characterized in that the plurality of drive beams are mounted in the central shaft joint through the drive beam joint, and when a staggered front and back occurs among the plurality of drive beams , the drive beam joint is capable of compensating the mismatch of the plurality of drive beams through rotational movement, such that the plurality of drive beams are simultaneously in contact with the drive beam joint all the time.

Further, the plurality of drive beams may include three inner drive beams and two outer drive beams.

Further, the plurality of drive beams may include two inner drive beams and two outer drive beams.

Further, the plurality of drive beams may include one inner drive beam and two outer drive beams.

Further, joint mounting features may be arranged at proximal ends of the plurality of drive beams, and the drive beam joint is connected to the drive beams through the joint mounting features.

Further, the drive beam joint is at least provided with a part of cylindrical segment, the central shaft joint is provided with a cylindrical cutout, a centre axis of the cylindrical cutout is oriented in a vertical direction, and the drive beam joint may be mounted in the cylindrical cutout and may rotate around the centre axis in the cutout hole.

Further, the joint mounting features of the drive beams may be cross-shaped mounting holes located at the proximal ends of the drive beams. Further, the joint mounting features of the drive beams may be I-shaped mounting holes located at the proximal ends of the drive beams. Further, the drive beam joint may be formed by cutting a part of a cylinder, such that the drive beam joint may be clamped in the cross-shaped or I-shaped mounting holes

Specifically, the cut part on the drive beam joint is divided into four parts which are symmetrically in upper, lower, far and near directions, and each cut part is cut through a direction parallel to the centre axis of the cylinder but not passing through the centre axis and a direction orthogonal to the centre axis but not passing through the centre axis, such that an upper side and a lower side of the drive beam joint are both knob-shaped, so defining a cylindrical segment located in a middle area, and knob sections located at an upper end and a lower end.

Further, the distance from top to bottom of the cross-shaped mounting hole is slightly greater than the maximum height of the drive beam joint, and the distance from the proximal end to the distal end of the cross-shaped mounting hole is slightly greater than the cylinder diameter of the central shaft joint; and the width of the knob section on the drive beam joint is basically consistent with the width from the proximal end to the distal end of the cross-shaped hole, the drive beam will generate a pulling force or a pushing force to the knob section when it moves back and forth, each drive beam will generate different forces when bending, and the drive beam joint rotates freely in the cylindrical cutout in the central shaft joint, such that when a staggered front and back occur among the plurality of drive beams, the drive beam joint is capable of compensating the mismatch through rotation movement, and the plurality of drive beams are simultaneously in contact with the drive beam joint.

Further, the central shaft joint further includes a first groove along a longitudinal axis of the central shaft joint, the first groove communicating with the cylindrical cutout, a distal end of the first groove being open and a proximal end of the first groove being closed; and when the drive beam joint is inserted into the cylindrical cutout of the centre joint, the proximal end of the drive beam is located at the closed proximal end of the first groove.

The adaptive drive beam structure provided by the present application is applied to rotating type devices, thus improving the stress of flexible drive beam in a device, ensuring that the plurality of drive beams are stressed uniformly in the movement process, reducing the deformation of a single piece drive beam and ensuring the movement smoothness of the drive beam structure in operation.

Further, the adaptive drive beam structure is applied to the rotating type endoscopic stapler, thus improving the stress of the flexible drive beam in the stapler, ensuring that the plurality of drive beams are stressed uniformly in the triggering process, reducing the deformation of the single piece drive beam and ensuring the movement smoothness of the drive beam structure in operation.

### BRIEF DESCRIPTION OF THE DRAWINGS

For clearer description of the technical solutions of the present application, the accompanying drawings required to describe the embodiments or the prior art are briefly described hereinafter. Obviously, the accompanying drawings to be described below are merely some embodiments of the present application, and a person of ordinary skill in the art may obtain other drawings according to those drawings without paying any creative effort.
FIG. 1 is a schematic diagram of bending and assembling of a stapler jaw;
FIG. 2 is a joint form of a rotating type stapler in the prior art;
FIG. 3 is a schematic diagram of a state when a staggered front and back occurs among drive beams of the rotating type stapler in the prior art;
FIG. 4 is a schematic diagram of assembling of drive beams according to the present application;
FIG. 5 is a structural schematic diagram when drive beams are in a normal state according to the present application; and
FIG. 6 is a structural schematic diagram when a staggered front and back occurs among drive beams according to the present invention.

In the drawings: 1, 5: outer drive beams; 2-4: inner drive beams; 6: drive beam joint; 7: central shaft joint.

### DETAILED DESCRIPTION OF THE EMBODIMENTS

The technical solutions in the embodiments of the present application are clearly and completely described below with reference to the accompanying drawings of the description. It will be apparent that the described embodiments are merely some rather than all of the embodiments of the present application. All other embodiments obtained by those of ordinary skill in the art based on the embodiments of the present application without creative efforts should fall within the protection scope of the present application.

The term "exemplary" is used herein to represent "serving as an example, an instance or illustration". Any implementation described herein as "exemplary" is not necessarily construed as more excellent than or superior to other implementations. Moreover, "proximal end", "near side" and "rear" refer to an end close to the operator, "distal end", "far side" and "front" refer to an end away from the operator, and the whole device is horizontally placed.

For the sake of brevity, the device of the present application is described by taking the articulating endoscopic stapler as an example. Those skilled in the art may understand that the adaptive drive beam structure provided by the present application is not limited to the articulating endoscopic stapler and is suitable for application to other rotating type devices.

The adaptive drive beam structure according to the present application includes a plurality of drive beams. There are five drive beams shown in FIG. 4. Those skilled in the art can easily understand that the number of the drive beams is only illustrative and may be selected as required. In the five drive beams in FIG. 4, outer drive beams 1, 5 are located on two sides, and inner drive beams 2, 3, 4 are located in the middle. The five drive beams 1-5 are mounted in the central shaft joint 7 through the drive beam joint 6. Specifically, joint mounting features are arranged at proximal ends of the drive beams 1-5, the drive beam joint 6 is fixedly connected to the joint mounting features, the drive beam joint 6 is at least provided with a part of cylindrical segment, the central shaft joint 7 is provided with a cylindrical cutout matched with the cylindrical segment of the drive beam joint 6, a centre axis of the cylindrical cutout is oriented in a vertical direction, and the drive beam joint 6 may be mounted in the cylindrical cutout and may rotate around the centre axis of the cuttout hole in the drive beam joint 6.

In FIG. 4, the joint mounting features of the drive beams are cross-shaped mounting holes located at the proximal ends of the drive beams, and the drive beam joint 6 is formed by cutting a part of a cylinder, such that the drive beam joint 6 may be clamped in the cross-shaped mounting holes. The cut part on the drive beam joint 6 is divided into four parts which are symmetrically in upper, lower, far and near directions, and each cut part is cut through in a direction parallel to the centre axis of the cylinder but not passing through the centre axis and a direction orthogonal to the centre axis but not passing through the centre axis, such that an upper side and a lower side of the drive beam joint 6 are both knob-shaped, so the drive beam joint 6 specifically defines a cylindrical segment located in a middle area, and knob sections located at an upper end and a lower end. The distance from top to bottom of the cross-shaped mounting holes on the drive beams is slightly greater than the maximum height of the drive beam joint 6, and the distance from the proximal end to the distal end of the cross-shaped mounting holes is slightly greater than the cylinder diameter of the central shaft joint 6. The width of the knob section on the drive beam joint 6 is basically consistent with the width from the proximal end to the distal end of the cross-shaped holes, so the drive beams will generate a pulling force or pushing force to the knob section when moving front and back, each drive beam generates different forces when the drive beams bend, and the drive beam joint 6 rotates freely in the cylindrical cutout in the central shaft joint 7, such that when a staggered front and back occurs among the five drive beams, the drive beam joint can compensate the mismatch through rotation movement, and the five drive beams 1-5 are simultaneously in contact with the drive beam joint 6.

Those skilled in the art can easily understand that the cross-shaped mounting holes at the proximal ends of the drive beams may be designed into other shapes, such as I shape, as long as the drive beam joint can be fixed on the drive beams and the rotation of the drive beam joint can cause a staggered front and back of each drive beam.

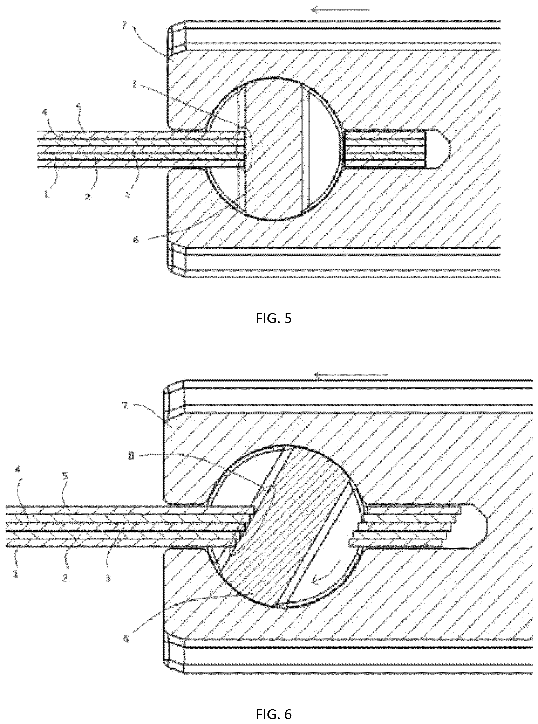

Referring to FIG. 4-6, the central shaft joint 7 further includes a first groove along a longitudinal axis of the central shaft joint, the first groove communicates with the cylindrical cutout, and the distal end of the first groove is open and the proximal end of the first groove is closed. When the drive beam joint 6 is inserted into the cylindrical cutout of the center joint 7, the proximal ends of the drive beams are located at the closed proximal end of the first groove, referring to FIG. 5 and FIG. 6.

Further referring to FIG. 5-6, when the central shaft joint 7 moves along an arrowhead direction, the central shaft joint 7 transmits an action force to the drive beam joint 6, and then the drive beam joint 6 transmits the force to the outer drive beams 1, 5 and the inner drive beams 2, 3, 4. When the outer drive beams 1, 5 and the inner drive beams 2, 3, 4 are in a normal state, the stress surfaces of the outer drive beams 1, 5 and the inner drive beams 2, 3, 4 align with each other and jointly bear the action force from the drive beam joint 6, as shown in FIG. 5; and when a staggered front and back occurs among the outer drive beams 1, 5 and the inner drive beams 2, 3, 4, the drive beam joint 6 will rotate in the central shaft joint 7 to adapt to and compensate the mismatch of the five drive beams, and at this time, the drive beam joint are still simultaneously in contact with the five drive beams, as shown in FIG. 6.

The adaptive drive beam structure according to the present application is applied to rotating type endoscopic stapler products, thus improving the stress of the flexible drive beams of the stapler, ensuring that the plurality of drive beams can be stressed uniformly in the triggering process, reducing the deformation of the single piece drive beam and ensuring the movement smoothness of the drive beam structure in operation.

The above description is of preferred embodiments of the present application. It should be noted that those skilled in the art may also make several improvements and modifications without departing from the principle of the present application which should be considered as the protection scope of the present application.

## Claims

1. An adaptive drive beam structure of a device, comprising a plurality of drive beams, a central shaft joint and a drive beam joint, **characterized in that** the plurality of drive beams are mounted in the central shaft joint through the drive beam joint, and when a staggered front and back occurs among the plurality of drive beams, the drive beam joint is capable of compensating the mismatch of the plurality of drive beams through self-adaptive movement, such that the plurality of drive beams are simultaneously in contact with the drive beam joint all the time.

2. The adaptive drive beam structure according to claim 1, **characterized in that** the drive beam joint is able to rotate relative to the central shaft joint to compensate the mismatch.

3. The adaptive drive beam structure according to claim 2, **characterized in that** joint mounting features are arranged at proximal ends of the plurality of drive beams, and the drive beam joint is connected to the drive beams through the joint mounting features.

4. The adaptive drive beam structure according to claim 3, **characterized in that** the drive beam joint is at least provided with a part of cylindrical segment, the central shaft joint is provided with a cylindrical cutout, a centre axis of the cylindrical cutout is oriented in a vertical direction, and the drive beam joint may be mounted in the cylindrical cutout and may rotate around the centre axis in the cutting hole.

5. The adaptive drive beam structure according to claim 4, **characterized in that** the joint mounting features of the drive beams are mounting holes located at the proximal ends of the drive beams.

6. The adaptive drive beam structure according to claim 5, **characterized in that** the drive beam joint is formed by cutting a part of a cylinder, such that the drive beam joint may be clamped in the mounting holes

7. The adaptive drive beam structure according to claim 6, **characterized in that** the cut part on the drive beam joint is divided into four parts which are symmetrically in upper, lower, far and near directions, and each cut part is cut through a direction parallel to the centre axis of the cylinder but not passing through the centre axis and a direction orthogonal to the centre axis but not passing through the centre axis, such that an upper side and a lower side of the drive beam joint are both knob-shaped, comprising a cylindrical segment located in a middle area, and knob sections located at an upper end and a lower end.

8. The adaptive drive beam structure according to claim 7, **characterized in that** the distance from top to bottom of the mounting hole is slightly greater than the maximum height of the drive beam joint, and the distance from the proximal end to the distal end of the mounting hole is slightly greater than the cylinder diameter of the central shaft joint; and the width of the knob section on the drive beam joint is basically consistent with the width from the proximal end to the distal end of the mounting hole, the drive beam will generate a pulling force or a pushing force to the knob section when it moves back and forth, each drive beam will generate different forces when bending, and the drive beam joint rotates freely in the cylindrical cutout in the central shaft joint, such that when a staggered front and back occurs among the plurality of drive beams, the drive beam joint is capable of compensating the mismatch through rotation movement, and the plurality of drive beams are simultaneously in contact with the drive beam joint.

9. The adaptive drive beam structure according to any one of claims 4 to 8, **characterized in that** the central shaft joint further comprises a first groove along a longitudinal axis of the central shaft joint, the first groove communicating with the cylindrical cutout, a distal end of the first groove being open and a proximal end of the first groove being closed; and when the drive beam joint is inserted into the cylindrical cutout of the center joint, the proximal end of the drive beam is located at the closed proximal end of the first groove.

10. The adaptive drive beam structure according to any one of claims 3 to 8, **characterized in that** the joint mounting features of the drive beams are cross-shaped mounting holes or I-shaped mounting holes located at the proximal ends of the drive beams.

11. A rotating type surgical device, comprising an adaptive drive beam structure.
